# EUROPEAN PATENT SPECIFICATION

(11) **EP 1 889 636 B1**
(45) Date of publication and mention of the grant of the patent: **08.06.2011**
(21) Application number: 05851071.0
(22) Date of filing: 24.05.2005
(51) Int. Cl.: A61M 16/04

(54) **ANTIREFLEXIVE ENDOTRACHEAL TUBE**
ANTIREFLEXIVER ENDOTRACHEALTUBUS
TUBE ENDOTRACHEAL ANTI-REFLEXE

(43) Date of publication of application: 20.02.2008
(73) Proprietor: Pervak, Vladimir Anatolievich, St. Petersburg 191040 (RU); Pervak, Konstantin Anatolievich, Vladivostok 690018 (RU)
(72) Inventor: Pervak, Vladimir Anatolievich, St. Petersburg 191040 (RU); Pervak, Konstantin Anatolievich, Vladivostok 690018 (RU)
(74) Representative: Ucinska, Julita
(86) International application number: PCT/RU2005/000285
(87) International publication number: WO 2006/126906

(56) References cited:
- GB-A- 1 060 629
- RU-C1- 2 150 300
- RU-C2- 2 219 965
- US-A- 4 417 576
- US-A- 4 693 243
- US-A- 5 146 916

## Description

### TECHNICAL FIELD

Present invention relates to medicine, namely to devices and instruments for injecting medical preparations into organism, in particular, to endotracheal tubes.

### BACKGROUND ART

Trendelenburg's tube is known (Difficulties in intubation of trachea/ Translated from English by M.N. Seleznev; edited by I.P. Lato, M. Rouzen.- M.: Medicine, 1989.-304 c., page 89-90).

The use of the Trendelenburg's tube for intubation of trachea causes pathophysiological responses of the human organism.

Dorrance's tube is known (Difficulties in intubation of trachea/ Translated from English by M.N. Seleznev; edited by I.P. Lato, M. Rouzen. - M.: Medicine, 1989. - 304 c., page 90).

However, the use of Dorrance's tube for intubation of trachea also causes pathophysiological responses of the human organism.

Also, Lanz's endotracheal tube is known (Difficulties in intubation of trachea / Translated from English by M.N. Seleznev; edited by I.P. Lato, M. Rouzen. - M.: Medicine. - M.: Medicine, 1989. - 304 c., page 97-99).

The use of Lanz's endotracheal tube for intubation permits to moderately reduce a level of pathophysiological responses of the human organism in view of the fact that Lanz's tube is provided with a regulating mechanism, which prevents excessive raise and reduction of pressure both in the cuff and across the tracheal wall.

Pervak's sealing cuff for an endotracheal tube is known (patent RU 2219965, IPC A 61M 16/04, published on December 27, 2003, bulletin No.36). The Pervak's sealing cuff contains an inflatable unit, perforated shell passing over the inflatable unit and a segment of the endotracheal tube adjacent to it on the opposite side of the proximal end, catheter of the inflatable unit and catheter of the perforated shell.

The use of the Pervak's sealing cuff enables to considerably reduce a level of pathophysiological changes of the human organism in responses to intubation of trachea, however, when using such highly toxic pharmaceutical substances as local anesthetics there is a risk of toxic responses of the human organism in connection with larges volumes of drugs, which accumulate in the space between the inflatable unit and the perforated shell passing over the inflatable unit.

There is known an intubation tube that has a device for drug irrigation (patent US 5,146,916, IPC A61M 16/04, A61M 25/10, issued September 15, 1992). The known intubation tube contains a tube body with proximal and distal ends, an inflatable cuff placed on its distal end, a catheter of the inflatable cuff, a device for drug irrigation made as a catheter for supply of drugs, two lateral ring ducts flush-mounted in ring grooves of the endotracheal tube body, one of them is more proximal and the second is more distal in respect to the inflatable cuff, and a shell passing over the inflatable cuff, as this takes place, there are many through holes in outer walls of lateral ring ducts and in the shell passing over the inflatable cuff.

A device with the features of the preamble of claim 1 is disclosed in document US 4693243. According to its function, technical essence and attained positive effect, this intubation tube with the device for drug irrigation is most close to present Pervak's antireflexive endotracheal tube.

However, when using the above-mentioned intubation tube that has the device for drug irrigation, if such highly toxic drugs as local anesthetics are applied, also there is a risk of toxic responses of the human organism in connection with large volumes of supplying drugs via through holes in walls of ring ducts and holes in the perforated shell passing over the inflatable unit.

When making the invention applied for, it was proposed to reduce a risk of toxic responses of the human organism when using such highly toxic drugs as local anesthetics during intubation. This was decided by reducing a total dose of injected drugs within the permissible limits, subject to a way for the reduction of pathophysiological responses of the human organism to a necessary extent.

### DISCLOSURE OF THE INVENTION

The above problems are decided, and the said positive effect is achieved by an antireflexive endotracheal tube, which contains a tracheal tube body with proximal and distal ends, an inflatable cuff placed on distal end of the tracheal tube body, a catheter of the inflatable cuff, a device for drug irrigation made as a catheter for supply of drugs, a lateral ring duct of the endotracheal tube body located more proximal in respect to the inflatable cuff, and a shell passing over the inflatable cuff. Also, a plurality of through holes are made in the outer wall of the lateral ring duct of the endotracheal tube body; the shell passing over the inflatable cuff is made as a lateral ring duct of the inflatable cuff placed at the center of the inflatable cuff in the plane, which is perpendicular to longitudinal axis of the endotracheal tube body; the antireflexive endotracheal tube is additionally provided with a longitudinal duct, which links a cavity of the lateral ring duct of the endotracheal tube body with a cavity of the lateral ring duct of the inflatable cuff; and a plurality of through holes are made in the outer wall of the lateral ling duct of the inflatable cuff.

Common essential features of the Pervak's antireflexive endotracheal tube in comparing with the above mentioned prototype such the intubation tube, which includes that device for drug irrigation are as follows:
- the antireflexive endotracheal tube;
- the body of the endotracheal tube with proximal and distal ends;
- the inflatable cuff;
- the inflatable cuff placed on its distal end;
- the catheter of the inflatable cuff;
- the device for drug irrigation,
- the device made as a catheter for supply of drugs, the lateral ring duct of the endotracheal tube body placed more proximal in respect to the inflatable cuff, and the shell passing over the inflatable cuff; and
- there are the through holes in the outer wall of the lateral ring duct of the endotracheal tube body.

Essential features which differ the Pervak's antireflexive endotracheal tube from the known intubation tube, including a device for drug irrigation, which are providing collectively with common essential features peculiar to the both tubes, and help to achieve the positive effect (considerable reduction of a risk of toxic responses of the human organism when using such highly toxic drugs during intubation of trachea as local anesthetics by reducing the total dose of introducing drugs within the permissible limits, subject to a way for the reduction of pathophysiological responses of the human organism to a necessary extent) in all cases covered by the requested scope of legal protection, are following features:
- the shell passing over the inflatable cuff is made as the lateral ring duct of the inflatable cuff;
- the shell placed at the center of the inflatable cuff of the antireflexive endotracheal tube in the plane, which is perpendicular to longitudinal axis of the endotracheal tube body;
- the antireflexive endotracheal tube is provided with the longitudinal duct;
- the longitudinal duct links the cavity of the lateral ring duct of the endotracheal tube body with the cavity of the lateral ring duct of the inflatable cuff; and
- there are through holes in the outer wall of the lateral ring duct of the inflatable cuff.

Making the shell passing over the inflatable cuff as the lateral ring duct of the inflatable cuff placed at the center of the inflatable cuff of the antireflexive endotracheal tube in the plane, which is perpendicular to longitudinal axis of the endotracheal tube body, as well as providing the antireflexive endotracheal tube with the longitudinal duct, which links the cavity of the lateral ring duct of the endotracheal tube body with the cavity of the lateral ring duct of the inflatable cuff, and making the through holes in the outer wall of the lateral ring duct of the inflatable cuff, permit to considerable reduce the total doses of introducing drugs within the permissible limits, and in doing so to considerably reduce a risk of toxic responses of the human organism when using such highly toxic drugs during intubation of trachea as local anesthetics, and at the same time to provide a way for the reduction of pathophysiological responses of the human organism to a necessary extent when supplying drugs in the area of structures localized above the fissure of glottis (root of tongue, arches of tonsils, uvula of soft palate, walls of pharynx etc.), i.e. permit to provide the achievement of the positive effect.

### BRIEF DESCRIPTION OF THE DRAWINGS

There is one figure of drawings which schematically illustrates the Pervak's antireflexive endotracheal tube.

### THE BEST EXAMPLE FOR CARRYING OUT THE INVENTION

An antireflexive endotracheal tube 1 includes a body 2 of the endotracheal tube with a proximal end 3 and a distal end 4, an inflatable cuff 5 placed on the distal end 4 of the body 2 of the endotracheal tube 1, a catheter 6 of the inflatable cuff 5 and a device of drug irrigation made as a catheter 7 for supply of drugs, a lateral ring duct 8 of the body 2 of the endotracheal tube 1, a lateral ring duct 9 of the inflatable cuff 5 and a longitudinal duct 10.

The lateral ring duct 8 of the body 2 of the endotracheal tube 1 is placed more proximal in respect to the inflatable cuff 5. In the outer wall of the lateral ring duct 8 of the body 2 of the endotracheal tube 1 there are through holes 11.

The lateral ring duct 9 of the inflatable cuff 5 is placed at the center of the inflatable cuff 5 of the endotracheal tube 1 in the plane which is perpendicular to the longitudinal axis of the body 2 of the endotracheal tube 1. In the outer wall of the lateral ring duct 9 of the inflatable cuff 5 there are through holes 12.

The longitudinal duct 10 links a cavity of the lateral ring duct 8 of the body 2 of the endotracheal tube 1 with a cavity of the lateral ring duct 9 of the inflatable cuff 5.

### INDUSTRIAL APPLICABILITY

Before using, the antireflexive endotracheal tube 1, the following should be tested: its physical configuration, air tightness of the inflatable cuff 5 and operationability of the device for drug irrigation by way of trial supply of drugs via the catheter 7 to the lateral ring duct 8 of the body 2 of the endotracheal tube 1 and then, via the lateral duct 10 to the lateral ring duct 9 of the inflatable cuff 5. Proper straying of drugs via the through holes 11 in the outer wall of the lateral ring duct 8 of the body 2 of the endotracheal tube 1 and via the through holes 12 of the lateral ring duct 9 of the inflatable cuff 5 testifies that the device of drug irrigation is serviceable.

Upon completion of positive testing, the distal end 4 of the endotracheal tube 1 is inserted into the trachea so that the lateral ring duct 9 of the inflatable cuff 5 can be more distal to vocal chords and the lateral ring duct 8 of the body 2 of the endotracheal tube 1 can be more proximal to the vocal chords.

After intubation of the trachea, the inflatable cuff 5 is inflated by supplying air with the help of syringe to the catheter 6, providing hermetic sealing of the trachea by this means. Necessary drugs are supplied in the catheter 7 by syringe to the lateral ring duct 8 of the body 2 of the endotracheal tube 1 and then, via the longitudinal duct 10 to the lateral ring duct 9 of the inflatable cuff 5. Drugs may contain local anesthetics, reparants of mucous membranes, antibiotics, antiseptics, hormones, ointment base and other components.

These drugs are injected via the through holes 11 of the lateral ring duct 8 of the body 2 of the endotracheal tube 1 as well as via the through holes 12 of the lateral ring duct 9 of the inflatable cuff 5, they come into contact with the mucous membrane of the trachea and supraligamentous structures (root of tongue, epiglottis) and provide a therapeutic effect.

Supply of drugs to the lateral ring duct 8 of the body 2 of the endotracheal tube 1 and to the lateral duct 9 of the inflatable cuff 5 may be carried out manually and automatically, as this takes place, and efficiency of the antireflexive endotracheal tube 1 in the automatic mode will be much more.

The use of the antireflexive endotracheal tube applied for enables to reduce a risk of toxic responses of the human organism when using such highly toxic drugs during intubation of trachea as local anesthetics by reducing the total dose of introducing drugs within the permissible limits, subject to a way for the reduction of laryngopharyngeal reflexes that cause hemodynamic and bronchoconstricting reactions, prophylaxis and treatment of infectious lesions over the area of contract of the inflatable cuff with mucosa, stimulation of regenerative properties of tracheal epithelium, prophylaxis and treatment of aseptic inflammatory processes, prevention or radical abatement of reflex phenomena arisen when the trachea is irritated by the endotracheal tube (local anesthetics), prevention or radical abatement of the development of tracheitis, both mild transitory (in anesthesiology), and severe pyonecrotic (prolonged artificial pulmonary ventilation in the course of intensive care), and ruling out of tracheostomy, which is inevitable at present in prolonged artificial pulmonary ventilation, or at least, postponement of tracheostomy.

The above-mentioned allows the conclusion that the use of the Pervak's antireflexive endotracheal tube applied to solve the problem and provide a positive effect.

The antireflexive endotracheal tube may be used successfully both as an endotracheal tube and a tracheostomic and endobronchial tube.

### LIST OF REFERENCE DESIGNATIONS AND NAMES OF ELEMENTS TO WHICH SUCH DESIGNATIONS ARE REFERRED

1 - an antireflexive endotracheal tube
2 - a body of the antireflexive endotracheal tube
3 - a proximal end of the antireflexive endotracheal tube
4 - a distal end of the antireflexive endotracheal tube
5 - an inflatable cuff
6 - a catheter of the inflatable cuff
7 - a catheter for supply of drugs
8 - a lateral ring duct of the endotracheal tube body
9 - a lateral ring duct of the inflatable cuff
10 - a longitudinal duct
11 - through holes of the lateral ring duct of the endotracheal tube body
12 - through holes of the lateral ring duct of the inflatable cuff

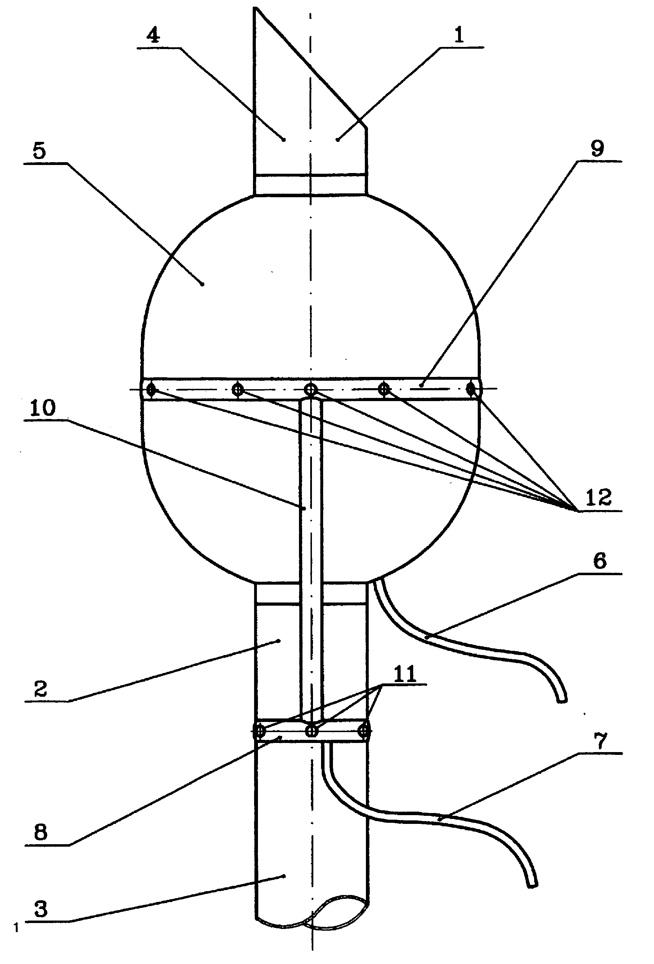

## Claims

1. An antireflexive endotracheal tube (1) including:
a body (2) of the endotracheal tube (1) having a proximal end (3) and a distal end (4),
an inflatable cuff (5) placed on the distal end (4),
a catheter (6) of the inflatable cuff (5),
a device for drug irrigation made as catheter (7) for supply of drugs;
a lateral ring duct (8) of the body (2) placed more proximal in respect to the inflatable cuff (5),
a plurality of through holes (11) formed in an outer wall of the lateral ring duct (8) of the body (2),
a shell passing over the inflatable cuff (5) and formed as a lateral ring duct (9) of the inflatable cuff (5) in a plane, which is perpendicular to a longitudinal axis of the body (2);
a longitudinal duct (10) linking a cavity of the lateral ring duct (9) of the inflatable cuff (5) with a cavity of the lateral ring duct (8) of the body (2); and
a plurality of through holes (12) formed in an outer wall of the lateral ring duct (9) of the inflatable cuff (5),
**characterizing in that** the lateral ring duct (9) of the inflatable cuff (5) is placed at the centre of the inflatable cuff(5).

## Patentansprüche

1. Ein antireflexiver Endotrachealtubus (1) einschließlich:
ein Körper (2) des Endotrachealtubus (1), mit einem proximalen Ende (3) und einem distalen Ende (4),
eine aufblasbare Manschette (5), die sich am distalen Ende (4) befindet,
ein Katheter (6) auf der aufblasbaren Manschette (5),
ein Gerät für die Spülung des Mittels gemacht wie ein Katheter (7) für Versorgung des Mittels,
ein seitlicher Ringkanal (8) des Körpers (2) platziert mehr proximal bezüglich der aufblasbaren Manschette (5),
die Vielzahl der Durchlöcher (11) formiert auf der Außenseite des seitlichen Ringkanales (8) des Körpers (2),
eine Schale umfassung die aufblasbare Manschette (5) und formiert wie der seitliche Ringkanal (9) der aufblasbare Manschette (5) auf der Fläche, die senkrecht zu den länglichen Achsen des Körpers (2) ist;
länglicher Kanal (10) verbindet Höhlung des seitlichen Ringkanales (9) der aufblasbaren Manschette (5) mit der Höhlung des seitlichen Ringkanales (8) des Körpers (2); und
die Vielzahl der Durchlöcher (12) formiert auf der Außenseite des seitlichen Ringkanales (9) der aufblasbaren Manschette (5),
**dadurch gekennzeichnet, dass** der seitliche Ringkanal (9) der aufblasbaren Manschette (5) in der Mitte der aufblasbaren Manschette (5) platziert ist.

## Revendications

1. Un tube endotrachéal anti-reflexe, (1) contenant:
un corps (2) du tube endotrachéal anti-reflexe (1) ayant une extrémité proximale (3) et une extrémité distale (4),
une manchette gonflable (5) située à l'extrémité distale (4),
un cathéter (6) de la manchette gonflable (5),
un dispositif pour la solution les médicaments, fait du cathéter (7) servant à doser les médicaments,
un anneau du conduit latéral (8) du corps (2) situé de manière plus proximale par rapport à la manchette gonflable (5),
une grande quantité d'orifices de passage (11) formée à la paroi extérieure de l'anneau du conduit latéral (8) du corps (2),
le corps alimentant la manchette gonflable (5) sous forme de l'anneau du conduit latéral (9) de la manchette gonflable (5) au plan, qui est perpendiculaire à l'axe longitudinale du corps (2);
un conduit longitudinal (10) liant l'orifice de l'anneau du conduit latéral (9) de la manchette gonflable (5) à l'orifice de l'anneau du conduit latéral (8) du corps (2); et
une grande quantité d'orifices de passage (12) formée à la paroi extérieure de l'anneau du conduit latéral (9) de la manchette gonflable (5),
**caractérisé en ce que** l'anneau du conduit latéral (9) de la manchette gonflable (5) est situé au milieu de la manchette gonflable (5).
